# EUROPÄISCHE PATENTSCHRIFT

(11) **EP 1 218 012 B1**
(45) Veröffentlichungstag und Bekanntmachung des Hinweises auf die Patenterteilung: **01.09.2004**
(21) Anmeldenummer: 00960220.2
(22) Anmeldetag: 21.09.2000
(51) Int. Cl.: A61K 31/557, A61P 9/00

(54) **VERWENDUNG VON ALPROSTADIL (PROSTAGLANDIN E1) ZUR HERSTELLUNG EINES ARZNEIMITTELS FÜR ANGIONEOGENESE**
USE OF ALPROSTADIL (PROSTAGLANDIN E1) FOR PRODUCING A MEDICAMENT FOR ANGIONEOGENESIS
UTILISATION D'ALPROSTADIL (PROSTAGLANDINE E1) POUR LA PRODUCTION D'UN MEDICAMENT DESTINE A LA NEOGENESE VASCULAIRE

(30) Priorität: 24.09.1999 AT 163599
(43) Veröffentlichungstag der Anmeldung: 03.07.2002
(73) Patentinhaber: Mehrabi, Mohammad Reza, A-1160 Wien (AT)
(72) Erfinder: Mehrabi, Mohammad Reza, A-1160 Wien (AT)
(74) Vertreter: Alge, Daniel, Mag. Dr. rer.nat.
(86) Internationale Anmeldenummer: PCT/AT2000/000252
(87) Internationale Veröffentlichungsnummer: WO 2001/022949

(56) Entgegenhaltungen:
- DIAZ-FLORES L ET AL: "Intense vascular sprouting from rat femoral vein induced by prostaglandins E1 and E2." ANATOMICAL RECORD, (1994 JAN) 238 (1) 68-76., XP001027418
- GULLINO P M: "Prostaglandins and gangliosides of tumor microenvironment: their role in angiogenesis." ACTA ONCOLOGICA, (1995) 34 (3) 439-41. REF: 15, XP001027467
- KOPF-MAIER P ET AL: "Influence of dispase and angiogenesis factors on the growth of human xenografts." JOURNAL OF CANCER RESEARCH AND CLINICAL ONCOLOGY, (1988) 114 (6) 547-52., XP001027417
- W. R. HIATT: "Current and future drug therapies for claudication" VASCULAR MEDICINE, Bd. 2, - 1997 Seiten 257-262, XP001027402

## Beschreibung

Die Erfindung bezieht sich auf die Verwendung von Alprostadil zur Herstellung eines Arzneimittels für Angioneogenese.

Cardiomyopathie (CMP) ist eine Krankheit, die zur Vergrößerung des Herzmuskels und zu Herzmuskelschwäche führt. Auf Grund der Herzmuskelschwäche kommt es zu einer verminderten Pumpfunktion und Auswurfaktion des Herzens. Die Pathogenese der dilatativen Cardiomyopathie ist nach wie vor unbekannt. Die ischämische Cardiomypathie kann auf Myokardinfarkt(e) zurückzuführen sein, da die abgestorbenen Herzmuskelareale durch Bindegewebe ersetzt werden. Dieser Herzmuskelersatz aus Bindegewebe kann jedoch keine kardialen Funktionen ausführen. In Folge kommt es zu einer Reduzierung der Herzmuskelfunktion und zur Wasseransammlung in der Lunge und in den unteren Extremitäten. Die Folgen sind starke Atemnot, Unbelastbarkeit und Müdigkeit.

Die Diagnose dieser Krankheit besteht auf Grund der Herzmuskelschwäche aus der typischen klinischen Symptomatik wie allgemeine Schwäche, körperliche Unbelastbarkeit, Atemnot unter Belstung und in Ruhe, rezidivierendem Lungenödem sowie Verschlechterung der Laborparameter mit pathologischem Anstieg der Nieren- und Leberparameter und einem gestörten Elektrolythaushalt. Echokardiographisch zeigt sich ein vergößertes Herz mit reduziertem Auswurf sowie ein inhomogenes Kontraktionsmuster. Patienten mit dilatativer CMP haben anamnestisch keinen Myocardinfarkt und bei der Koronarangiographie wird keine (wirksame) Koronarsklerose festgestellt.

Alprostadil (PGE1) ist ein Medikament, das ursprünglich wegen seines guten vasodilatatorischen Effekts bei der Behandlung von Neugeborenen mit (anatomischen) kardio-pulmonalen Missbildungen Verwendung fand. Weiterer eingeschränkter Einsatz von Alprostadil (PGE1) ist die chronisch-erektile Dysfunktion.

In der EP 0 153 858 A2 wird de Verwendung von Prostaglandinen (darunter auch Prostaglandin E1) zur Behandlung von multiplen Organschäden, akuten respiratorischem Distress-Syndrom (ARDS; Schocklunge), Schocktrauma oder Sepsis beschrieben.

Forth et al. ("Allgemeine und spezielle Pharmakologie und Toxikologie", 7.Aufl. (1996), S.344, Spalte 1, 2. Absatz) geben ausschließlich den bekannten vasodilatatorischen Effekt von Prostaglandin E1 wieder, in dem die hämodynamischen Parameter zu dieser bislang einzigen arzneimittelrechtlich zugelassenen Indikation beschrieben sind.

Rabinowitz et al. (Am.J.Ther. 4 (11/12) (1997), S.353-358) beschreiben die hämodynamische Wirkung von Prostaglandin E1 auf Patienten mit koronarer Herzkrankheit mit stabiler und instabiler Angina Pectoris, die einer Intervention mit PTCA (Herzkathteter mit Ballondilatation) oder Bypass-Operation unterzogen wurden oder einen akuten Myokardinfarkt erlitten hatten. Weiters wird eine Erhöhung der Hauttemperatur nach der Anwendung von Prostaglandin E1 bei Patienten mit peripher arterieller Verschlusskrankheit festgestellt.

Wimmer et al. (Jpn Heart J. 40(3) (1999), S.321,334) berichten über die Expression neurohumoraler Mediatoren bei Prostalgandin E1-Therapie im Vergleich zu Dobutamin bei Patienten mit chronischer Herzinsuffizien. Diese Studie zeigt einen besseren hämodynamischen Effekt der Prostaglandin E1 behandelten Gruppe verglichen mit der mit Dobutamin behandelten Gruppe. Weiters wurde die Nierenfunktion an Hand der Paraaminohippurat-Clearance sowie der Iothalamat-Clearance untersucht, wobei die Patienten der Prostaglandin-Gruppe bessere Nierenwerte zeigten.

Im Artikel von Meyer et al. (Anesth.Analg. 86(1998), S.753-358) wurde die hämodynamische Wirkung von Prostanglandin E1 in inhalativer Form kombiniert mit NO-Gas bei Patienten mit multiplem Organversagen evaluiert, wobei sich eine Verbesserung dieser Parameter bei der angewendeten Therapieform zeigte.

Sterling et al. (Liv.Transp.Surg. 4(5) (1998), S.424-431) offenbaren die Behandlung von Patienten mit akutem Leberversagen, wobei es als unklar dargestellt wird, ob Prostaglandin E1 irgendeine Wirkung aufweist, wenn diese Therapie innerhalb von 10 Tagen begonnen wird. Es wird jedoch darauf hingewiesen, dass die Ergebnisse zeigten, dass Prostaglandin E1 keine Wirkung zeigte, wenn die Behandlung erst 10 Tage nach Einsetzen der klinischen Symptome begonnen wurde. Aus der Sicht der Autoren ist Prostaglandin E1 demgemäß zur Behandlung von akutem Leberversagen (fulminant hepatic failure (FHF)) nicht geeignet.

Iwata et al. (J.Gast.Hepatol. 14(1999), S.634-641) beschreiben Tierversuche, bei welchen Prostaglandin E1 direkt in Lebervenen infundiert wurde. Dabei zeigte die Prostaglandin E1 behandelte Gruppe serologisch mehr Leukozyten. Es wird daher geschlossen, dass hepatische Perfusion mit Prostaglandin E1 Mikrozirkulationsschäden, die mit Ischämie und Reperfusion durch Inhibierung von Leukozyten-Endothelium- Wechselwirkungen bedingt sind, behandelt werden können.

Diaz-Flores et al. (Anatomical Record 238(1994), S. 68-76) untersuchten das Gefäßwachstum in Tumoren anhand von Substanzen, welche vom Tumor selbst für sein eigenes Wachstum produziert werden (darunter gehören auch Prostaglandine), untersucht. Nachdem das Tumorwachstum einen autonomen Prozess mit einer völlig autonomen, durch den Tumor selbst induzierten und gesteuerten Angiogenese, darstellt, wurde in dieser Studie keine neue Wirkung (de novo Bildung von Gefäßen) durch PGE nachgewiesen, sondern lediglich ein ohnehin schon primär vorhandenes Tumorgefäßwachstum, durch die nachträgliche Zugabe von Gangliosiden und Prostaglandinen weiter beeinflusst.

Gullino (Acta Ocologica 34(1995), S. 439-441) beschreibt ebenfalls eine onkologische Studie an Mäusen, wo wiederum das Wachstumsverhalten eines Tumors untersucht wird. Hierbei wurde eine Reihe pharmakologisch völlig unterschiedlicher Substanzen (darunter auch das von Tumoren selbst produzierte Prostaglandin E) in hoher Dosierung appliziert, die aber alle zum gleichen Ergebnis, einem beschleunigten Tumorwachstum bei Mäusen mit fehlender Immunabwehr (und damit fehlender körpereigener Tumorbekämpfung) führt.

Kopf-Maier et al. (J Cancer Res Clin Oncology 114(1988), S. 547-552) untersuchen einen möglichen Effekt von PGE1 und PGE2 bezüglich einer Gefäßneubildung, der von Venen ausgeht. In dieser Studie wird durch eine, durch exogene Zufuhr von Prostaglandinen in toxischer Konzentration eine Gewebeschädigung mit nachfolgender Narbenabheilung indiziert, weshalb sich die von der Vene ausgehenden neu gebildeten Gefäße auch nach Beendigung des Abheilungsprozesses (2 Wochen) wieder zurückgebildet haben.

Aufgabe der vorliegenden Erfindung war eine neue Verwendung von Alprostadil (Prostaglandin E1) zur Verfügung zu stellen, die nicht auf dessen vasodilatatorischer Wirkung beruht.

Diese Aufgabe wurde durch die Verwendung von Alprostadil zur Herstellung eines Arzneimittels für Angioneogenese im Rahmen der Behandlung chronischer Herzinsuffizienz und/oder Kardiomyopathie gelöst. Weitere spezielle Anwendungen der erfindungsgemäßen Angioneogenese sind in den Ansprüchen 2-7 beschrieben. Der erfindungsgemäß zur Verfügung gestellte Effekt von Alprostadil auf die Angioneogenese im Rahmen der Behandlung chronischer Herzinsuffizienz und/oder Kardiomyopathie war in Anbetracht der bekannten Wirkungen von Prostaglandin E1 völlig überraschend. Es zeigte sich, dass Patienten mit chronischer Herzinsuffizienz ebenso erfolgreich behandelt werden konnten wie Patienten mit fortgeschrittenen peripheren arteriellen Verschlusskrankheiten, diabetische Angiopathie sowie systemischen Lungenerkrankungen. Weiters konnten auch abgestorbene Herzareale, insbesondere nach einem Herzinfarkt, revitalisiert werden. Dies ist jedenfalls darauf zurückzuführen, dass andere funktionelle Prozesse als Vasodilatation für diesen Effekt verantwortlich sind, z.B. eine effizientere Sauerstoffversorgung der Myozyten durch eine vermehrte Blutzufuhr.

Im Unterschied zur gängigen Lehrmeinung führt die erfindungsgemäß angewendete Alprostadil-Therapie offensichtlich zu:
a) Reduktion des pathologischen Fibrosegrades,
b) Regression der Muskelhypertrophie bei chronischer Herzinsuffizienz,
c) Erhöhung des Herz-Index bei chronischer Herzinsuffizienz;
d) Erhöhung der Auswurfaktion bei chronischer Herzinsuffizienz;
e) Ausschwemmung von Lungenödemen bei chronischer Herzinsuffizienz;
e) Senkung der erhöhten Drücke im kleinen Kreislauf (Lungenkreislauf) bei chronischer Herzinsuffizienz;
f) Stabilisierung des Blutdrucks bei chronischer Herzinsuffizienz;
g) Verbesserung der Atemnot und Rückgang des NYHA-Stadiums;
h) Abbau der Muskelhypertrophie bei CMP und Hypertonie-bedingte Kardiomyopathie.

Die Applikation von PGE1 erfolgt erfindungsgemäß bevorzugt durch (intravenöse) Infusion. Je nach Patient oder Krankheitssituation kann die Applikation aber auch vorzugsweise intracoronar erfolgen. Gleichfalls angezeigt können besonders eine epicardiale Applikation im Perikardraum, eine lokale Applikation unter Zuhilfenahme von Applikationsballonen, eine Applikation in Coronarvenen unter Zuhilfenahme von retrograden Perfusionstechniken sowie eine transmyocardiale Applikation unter Zuhilfenahme von Laser, Hochfrequenzablation und/oder Injektionsnadeln sein.

Die Erfindung wird anhand der nachfolgenden Beispiele und der Zeichnungsfiguren, auf die sie jedoch nicht beschränkt sein soll, näher erläutert.

Es zeigen:
Fig. 1 CD-34-positive Kapillaren im Subepicardium (Subepi), Myocardium (Myocard) und Subendocardium (Subendo) bei Alprostadil (PGE1)-behandelten Patienten im Vergleich zur Kontrollgruppe;
Fig.2 vWF-positive Kapillaren im Subepicardium (Subepi), Myocardium (Myocard) und Subendocardium (Subendo) bei Al-Kontrollgruppe;
Fig.4 VEGF-positive Kapillaren im Subepicardium (Subepi), Myocardium (Myocard) und Subendocardium (Subendo) bei Alprostadil (PGE 1)-behandelten Patienten im Vergleich zur Kontrollgruppe;
Fig.5 den Fibrosegrad bei Alprostadil (PGE 1)-behandelten Patienten im Vergleich zur Kontrollgruppe;
Fig. 6 repräsentative Bilder von PGE1 behandelten Patienten (6A: CD-34-positive Kapillaren; 6B: vwF-positive Kapillaren, 6C: MIB-1-positive Kapillaren, 6d: VEGF-positive Kapillaren (die Pfeile markieren jeweils positive Kapillaren; Fig.6A-C sind mit 1000facher Vergrößerung aufgenommen; Fig.6D mit 400facher Vergrößerung));

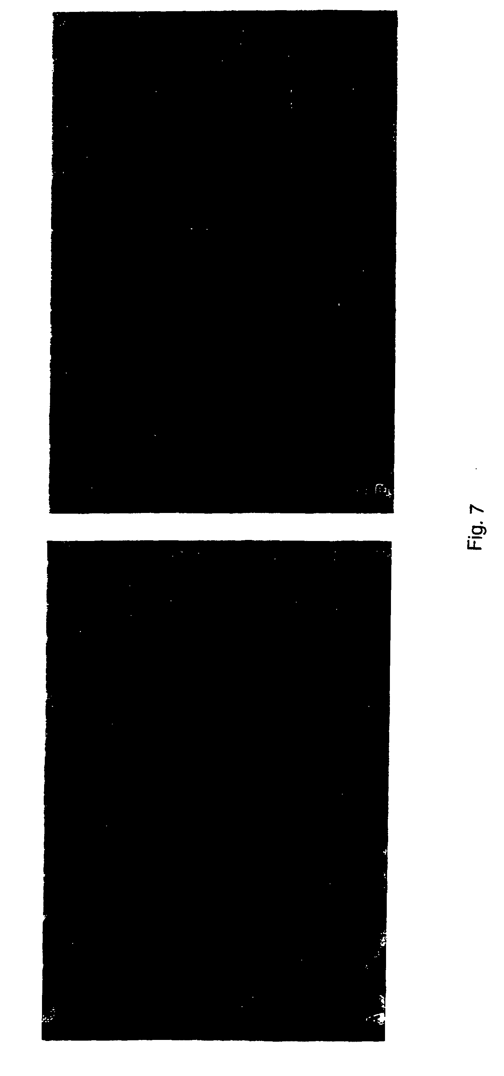

Fig.7 eine Sirius-Rot-Färbung (Fibrosegehalt) von einem Patienten nach PGE1-Therapie (Fig.7A) und einem Patienten ohne PGE1-Therapie (Fig.7B);
Fig.8 CD34 (behandelt (Fig.8A) und unbehandelt (Fig.8B)), vwF (behandelt (Fig.8C) und unbehandelt (Fig.8D), VEGF (behandelt (Fig.8E) und unbehandelt (Fig.8F)) sowie MIB-1 (behandelt (Fig.8G) und unbehandelt (Fig.8H).

### Beispiele:

### 1. Klinische Studie an Patienten mit CMP

Patienten mit CMP wurden für eine Herztranslation (HTX) angemeldet und medikamentös mit ACE-Hemmern, β-Blocker, Diuretika und Digitalis behandelt.

In einer vorangegangenen Studie erhielten mehrere Patienten mit Kardiomyopathie, darunter 9 Patienten mit dilatativer CMP vor einer HTX zusätzlich eine Alprostadil (PGE 1)-Infusionstherapie.

Die Einschlusskriterien für die Alprostadil (PGE 1)-Studie waren:
a. Patienten waren in ihrer täglichen Aktivität schwerwiegend eingeschränkt, obwohl sie eine maximale oralmedikamentöse Therapie mit ACE-Hemmer (Angiotensin-Converting-Enzym-Antagonisten), Diuretika und Digitalis bekamen.
b. Die Hämodynamik der Patienten zeigte einen niedrigen Herz-Index (< 2,5 L/min/m2) und einen höheren PCWP (pulmonory capillary wedge pressure) > 20 mm HG).
c. CMP-Patienten, die einen erhöhten peripheren Gefäßwiderstand aufweisen.

Alprostadil (PGE1) gehört nicht zur Standardtherapie der Kariomyopathie, wurde jedoch wegen seiner guten vasodilatatorischen Wirkung bei Patienten mit Kardiomyopathie und erhöhtem peripheren Gefäßwiderstand als adjuvante Therapie zur Entlastung des Herzzens bis zur Durchführung einer Herztransplantation verwendet.

Die Infusionstherapie erfolgte unter laufender Messung der hämodynamischen Parameter. Es zeigte sich eine deutliche Verbesserung der hämodynamischen Parameter im Vergleich zu den Ausgangswerten bei gleichzeitiger Verbesserung der klinischen Symptomatik. In dieser klinisch-experimentellen Studie wurde die Alprostadil (PGE1)-Infusionstherapie bei Patienten im Endstadium der Herizinsuffizienz im Sinne einer adjuvanten Therapie bis zur HTX verwendet. Interessanterweise wurde bei dieser Gruppe von Patienten eine deutliche Verbesserung des klinischen Zustands mit Steigerung der Auswurfraktion und des Herzminutenvolumens verbunden mit einer Reduzierung des NYHA-Stadiums sowie der pathologischen Drücke im Lungenkreislauf diagnostiziert.

Die Alprostadil (PGE1)-Infusionen erfolgten über einen zentralen Rechtsherzkatheter (Hickman-Katheter) mit einer anfänglichen Dosis von 2,5 ng/kg/min, die dann bis zur maximal tolerierten Dosis (MTD) gesteigert wurde. MTD war die Dosis, bei der eine der folgenden Nebenwirkungen auftraten: Muskelschmerzen, Knochenschmerzen, Blutdruckabfall, Übelkeit, Erbrechen, Durchfall, Kopfschmerzen oder andere Nebvenwirkungen. Unter der MTD (29 ± 1 ng/kg/min) wurden die hämodynamischen Parameter dokumntiert. Die MTD wurde in den folgenden 12 Stunden halbiert und nach der Stabilisierung der Hämodynamik wurde die Therapie mit einer tragbaren Pumpe zu Hause fortgesetzt.

Um die Frage zu beantworten, ob nun die Alprostadil (PGE1)-Infusionstherapie mit Neovakularisation einhergeht, wurden nach der HTX die explantierten Herzen von Patienten mit vorangegangener Alprostadil (PGE 1)-Infusionstherapie und von Patienten ohne alprostadil (PGE 1)-Therapie immunhistochemisch untersucht und die Kappilardichte verglichen.

Jedes explantierte Herz wurde in drei gleich große Stücke, nämlich in Spitze, mittleren und basalen Teil geteilt. Die Schnittführung erfolgte transmural durch den mtitleren Teil des linken Ventrikels. Das Gewebe für immunohistochemische Analysen wurde sofort nach der Entnahme in Formaldehyd nach der üblichen Methode konserviert.

Die Kapillardichte wurde getrennt im Subepicardium, Myocardium und dem Subendocardium bestimmt. Die Bestimmung der Kapillardichte 1,mm² wurde getrennt anhand von anti-CD34, von Willebrand-Faktor (vwf), vascular-endothelial-growth-factor (VEGF) , anti-Ki 67 (MIB 1) und Sirius Rot, immunhistochemisch gefärbter Paraffinschnitte durchgeführt.

### Ergebnisse

Die hämodynamischen Parameter vor der Alprostadil (PGE 1) Therapie waren deutlich schlechter in der Alprostadil (PGE 1)-Gruppe als in der Kontrollgruppe bei einem vergleichbaren mittleren Alter (52,33 ± 11,89 versus 49,88 ± 20,42 Jahre, p = 0,76). Die Patienten der Alprostadil (PGE 1 )-Therapie hatten einen bedeutend niedrigeren cardiac index (CI) (1,64 ± 0,29 L/min/m2 gegenüber 2,39 ± 0,26 L/min/m2, p <0,0001), und einen höheren pulmonary vascular resistenz index (PVRI) (605,11 ± 149,80 dyn x Sek x cm-5 x m-2 gegenüber 372,75 ± 84,63 dyn x Sek x cm-5 x m-2, p = 0,0015). Beide Gruppen hatten abgesehen von der Alprostadil (PGE 1)-Therapie eine vergleichbare medikamentöse Behandlung.

Die Kapillardichte im Subepicardium, Myocardium und Subendocardium von transmuralen Schnitten wurde quantitativ bestimmt und verglichen. Patienten, die eine Alprostadil (PGE 1)- Infusionen bekamen, hatten deutlich mehr Kapillaren (p <0,001) pro mm² als die Kontrollgruppe. Die Kapillardichte im Subepicardium, Myocardium und Subendocardium von der Kontrollgruppe betrug 608,56 ± 91,32 Kapillaren/mm² (sEpi), 542,44 ± 197,20 Kapillaren/mm² (sEndo), und 452,22 - 101,99 Kapillaren/mm² (Myo) in den drei transmuralen-Arealen. Alprostadil (PGE 1)-behandelte Patienten hatten im Vergleich dazu mit 1168,11 ± 165,04 Kapillaren/mm² (sEpi), 1066,00 ± 94,63 Kapillaren/mm² (SEndo), und 974,56 ± 87,12 Kapillaren/mm² (Myocard), ungefähr 50% mehr Kapillaren als die Kontrollgruppe.
Untersuchung hinsichtlich spezifischer immunohistochemischer Kapillarmarker:

### CD 34, endothelialer Zellmarker

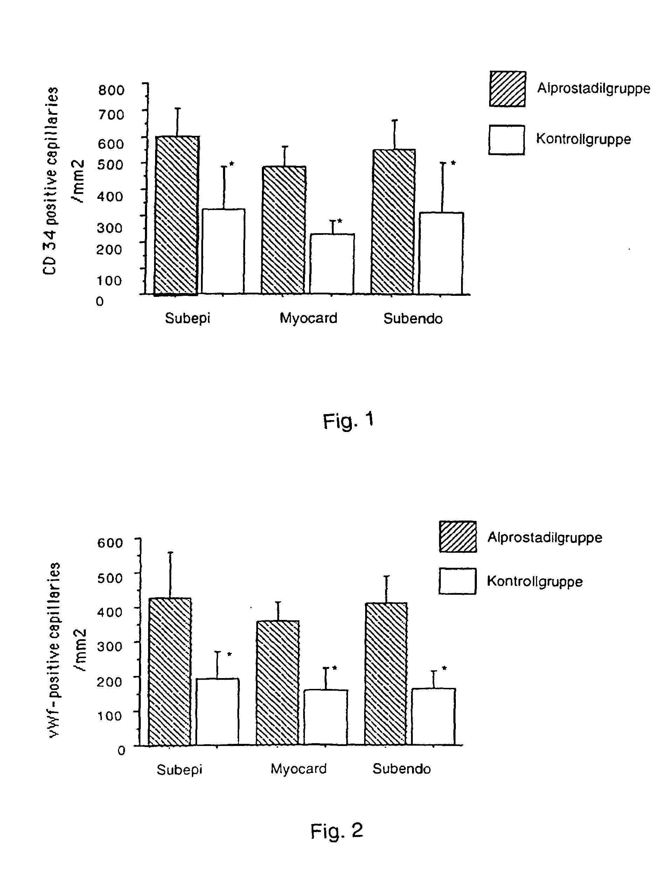

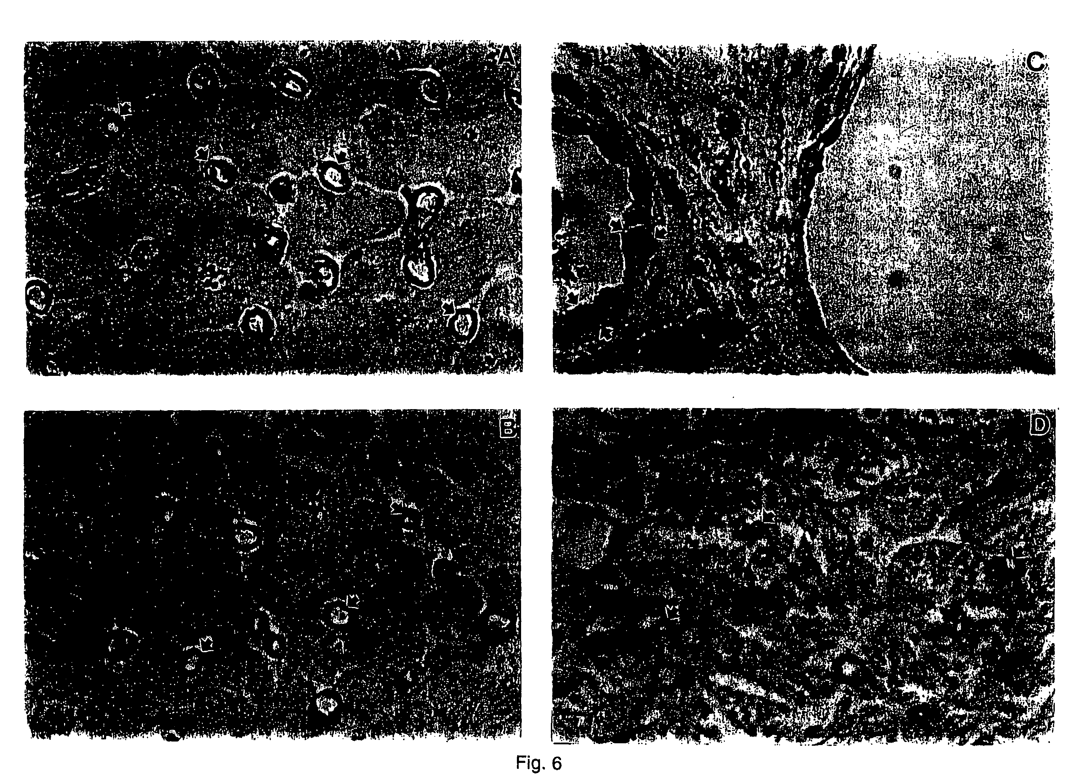

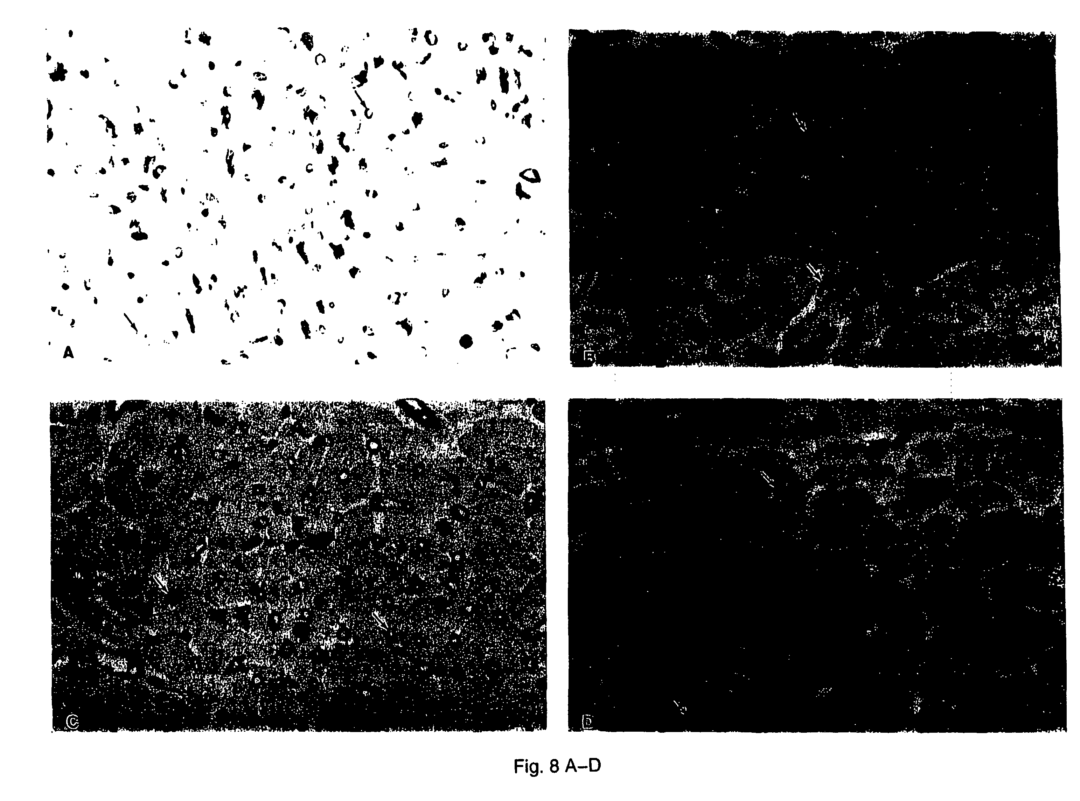

CD 34 wird von allen Endothelzellen im normalen Gewebe exprimiert. CD 34 wird auch zum Nachweis von Endothel bzw. -zellen benutzt. Patienten nach einer Alprostadil (PGE 1)-Therapie wiesen deutlich mehr anti-CD34 reaktives Endothel (Fig.1) auf als die Kontrollgruppe (Subepicardium: 599,22 ± 107,17 mm² gegenüber 322,89 ± 160,64 mm²-Zellen, p <0,001; Myocardium: 482,11 ± 79,86 mm² vs. 227,22 ± 49,30 mm² , p<0.0001; Subendocardium: 482,11 ± 79,86 mm² gegenüber 227,22 ± 49,30 mm², p <0,0001); Subendocardium: 551,67 ± 107.74 mm² gegenüber 308,56 ± 193.86 mm², p < 0,01). Die Ergebnisse sind auch in Fig.6A, 8A und 8B dargestellt.

### Faktor VIII Related Antigen, Von Willebrand Faktor (vWf)

Als zweiter pan-Endothelzell-spezifischer Marker wurde Faktor VIII immunhistochemisch untersucht. Die Herzen von CMP-Patienten nach Alprostadil(PGE 1)-Infusionstherapie zeigten bedeutend mehr anti-vWf reaktive Endothelien (Abb.2) verglichen mit der Kontrollgruppe (Subepicardium: 425,56 ± 134.17 mm² gegenüber 192,22 ± 77,88 mm²-Zellen, p <0,001; Myocardium: 360,00 ± 52,31 mm² gegenüber 159,89 ± 61.00 mm² p <0,0001; Subendocardium: 408,00 ± 80,00 mm² gegenüber 163,89 ± 47,52 mm², p <0,0001). Die Ergebnisse sind auch in Fig.6B, 8C und 8D dargestellt.

### MIB-1 (Proliferationsmarker)

MIB-1 (anti-Ki67), ist ein Antikörper, der nur mit Zellen reagiert, die nicht in der GO-Phase des Zellzyklus sind und wird üblicher Weise als Mitose- oder Proliferationsmarker eingesetzt. Er zeigt eine charakteristische Reaktion bei mitotischen Zellen.

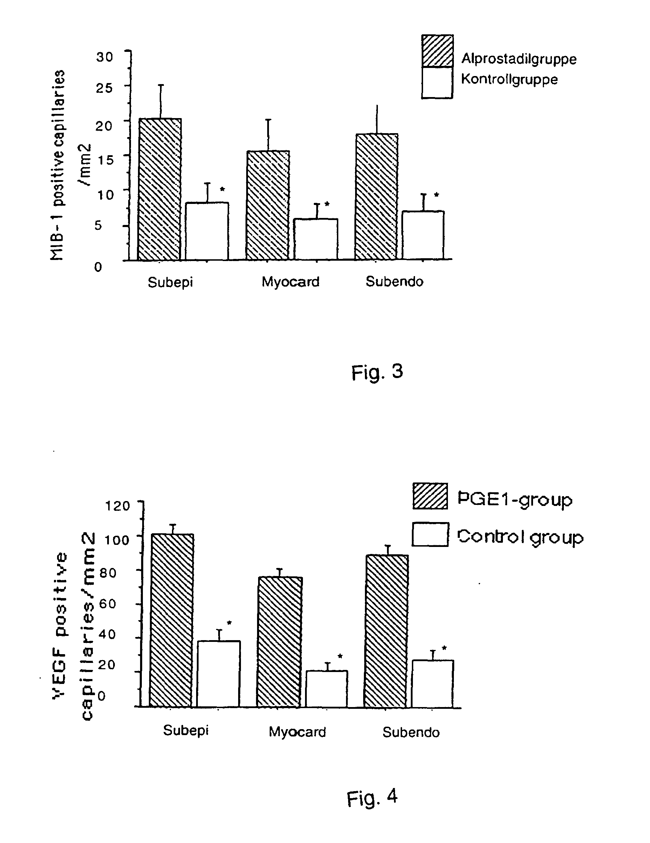

Bei der Gruppe der Patienten mit Alprostadil(PGE 1)-Infusionstherapie war die Anzahl der MIB-1 positiven Zellen (Fig.3) in allen Schichten des Herzmuskels deutlich höher verglichen mit der Kontrollgruppe: (Subepicardium: 20,22 ± 4,87 pro mm² gegenüber 8,11 ± 2,67 pro mm², p <0,0001; Myocardium: 15,44 ± 4,64 pro mm² gegenüber 5,78 ± 2,11 pro mm², p <0,0001; Subendocardium: 17,84 ± 4,23 pro mm² gegenüber 6,89 ± 2,21 pro mm2). Die Ergebnisse sind auch in Fig.6C, 8G und 8H dargestellt.

### Anti-VEGF-Immunreaktivität von Kapillaren

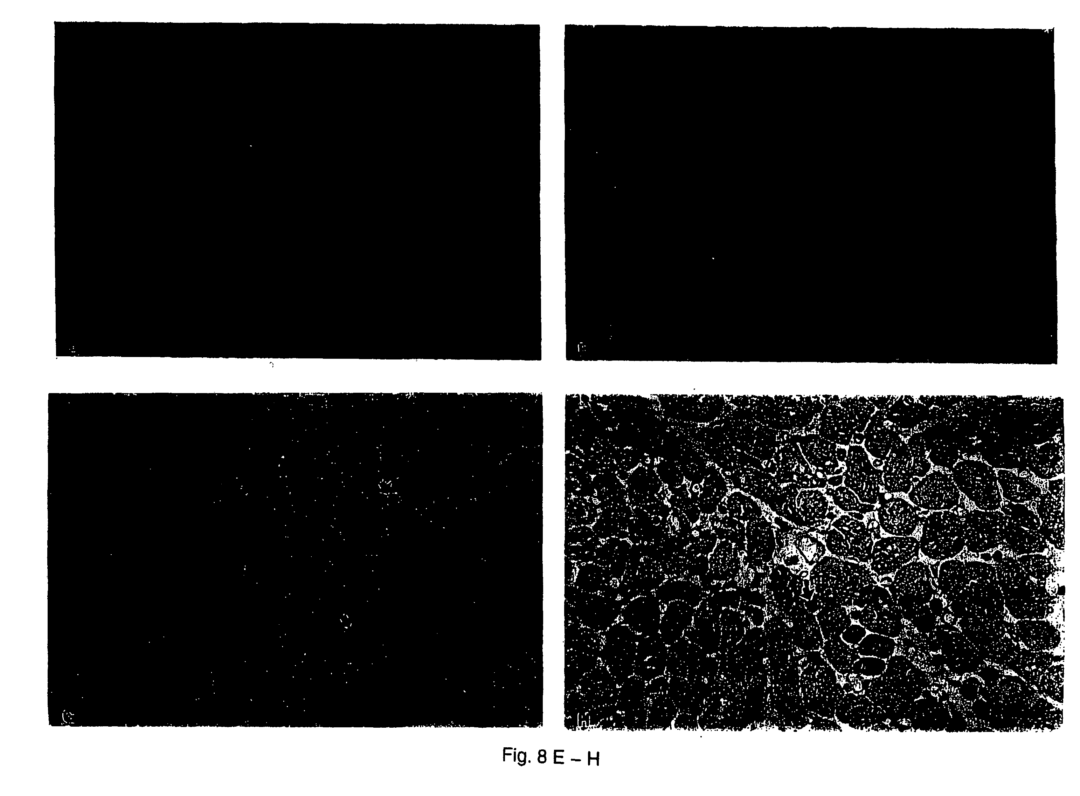

VEGF ist beschrieben als spezifischer Wachstumsfaktor für Endothelzellen. Alprostadil (PGE 1)-behandelte Patienten zeigten bedeutend mehr VEGF-positive Kapillaren/mm² als die Kontrollgruppe (Fig.4) in allen drei untersuchten Abschnittsebenen (Subepicardium: 101,2 ± 5,5/mm² gegenüber 38.1 ± 7,2-VEGF-positive Zellen/mm², p <0,0001; Myocardium 76,2 ± 4,9/mm² gegenüber 20,6 ± 4,9/mm², p <0,0001; Subendocardium: 89,1 ± 5,7/mm² gegenüber 27,8 ± 5,1/mm², p <0,0001). Die Ergebnisse sind auch in Fig.6D, 8E und 8F dargestellt.

Damit ist bewiesen, dass - im Unterschied zur gängigen Lehrmeinung - die Alprostadil (PGE 1)-Therapie zu einer Neovaskularisation des Herzens bei Patienten mit CMP führt. Diese Eigenschaften sprechen für die Alprostadil (PGE 1)-Therapie als Standardtherapie bei Patienten mit chronischer Herzinsuffizienz und Kardiomyopathie.

### Fibrose und Muskel-Masse (Sirius-Rot-Färbung)

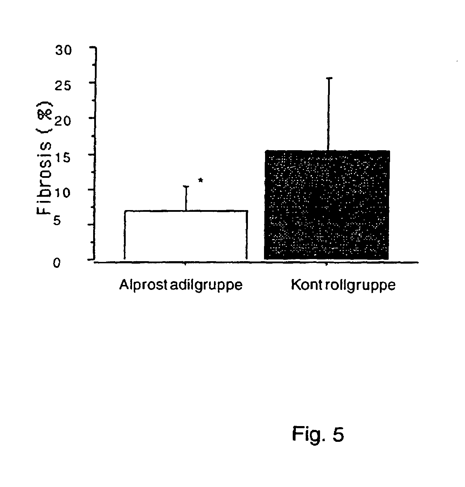

Die Bestimmung des Fibrosegrads und des Herzmuskelanteils bei Alprostadil (PGE 1)-behandelten Patienten (Fig.5) gegenüber der Kontrollgruppe zeigte, dass die Alprostadil (PGE1)- Infusionstherapie den Fibrosegrad bei Patienten mit dilatativer CMP deutlich verringert (15,35 ± 10,32% gegenüber 6,89 ± 3,59%, p <0,05, bei einem vergleichbaren Herzmuskelanteil (72,69 ± 5,25% gegenüber 68,75 ± 6,23%).

### Hypertrophie:

In PGE1-behandelten Patienten wurden Muskelzahl/mm² mit prozentuellem Muskelanteil/mm² bestimmt und mit unbehandelten Patienten und gesunden Kontrollen verglichen. Die Ergebnisse sind in der nachfolgenden Tabelle angegeben.

| | ***Muskelzahl*/*mm***^{***2***} | ***% Muskelanteil*/*mm***^{***2***} |
|---|---|---|
| PGE1-behandelte Patienten | 982,68 ± 141,43 | 74,9 ± 3,7 |
| unbehandelte Patienten | 865,35 ± 160,64 | 69,7 ± 2,8 |
| Kontrollen | 1265,04 ± 59,82 | 79,5 ± 2,5 |

## Patentansprüche

1. Verwendung von Alprostadil zur Herstellung eines Arzneimittels für Angioneogenese im Rahmen der Behandlung chronischer Herzinsuffizienz und/oder Kardiomyopathie.

2. Verwendung nach Anspruch 1, **dadurch gekennzeichnet, dass** die Angioneogenese zur Senkung des Fibrosegrades verwendet wird.

3. Verwendung nach Anspruch 1 oder 2, **dadurch gekennzeichnet, dass** die Angioneogenese zur Regression der Hypertrophie verwendet wird.

4. Verwendung nach einem der Ansprüche 1 bis 3, **dadurch gekennzeichnet, dass** die Angioneogenese zur Revitalisierung abgestorbener Herzareale, insbesondere nach einem Herzinfarkt, verwendet wird.

5. Verwendung nach einem der Ansprüche 1 bis 4, **dadurch gekennzeichnet, dass** die Angioneogenese zur Behandlung von fortgeschrittenen peripheren arteriellen Verschlusskrankheiten verwendet wird.

6. Verwendung nach einem der Ansprüche 1 bis 5, **dadurch gekennzeichnet, dass** die Angioneogenese zur Behandlung von diabetischer Angiopathie verwendet wird.

7. Verwendung nach einem der Ansprüche 1 bis 6, **dadurch gekennzeichnet, dass** die Angioneogenese zur Behandlung von systemischen Lungenerkrankungen, insbesondere Lungenfibrose, verwendet wird.

## Claims

1. The use of Alprostadil for preparing a medicament for angioneogenesis within the scope of the treatment of chronic cardiac insufficiency and/or cardiomyopathy.

2. The use according to claim 1, **characterised in that** the angioneogenesis is used to lower the degree of fibrosis.

3. The use according to claim 1 or 2, **characterised in that** the angioneogenesis is used for the regression of hypertrophy.

4. The use according to any one of claims 1 to 3, **characterised in that** the angioneogenesis is used for revitalizing necrotic heart regions, in particular following cardiac infarction.

5. The use according to any one of claims 1 to 4, **characterised in that** the angioneogenesis is used for treating advanced-stage peripheral arterial occlusive diseases.

6. The use according to any one of claims 1 to 5, **characterised in that** the angioneogenesis is used for treating diabetic angiopathy.

7. The use according to any one of claims 1 to 6, **characterised in that** the angioneogenesis is used for treating systemic lung diseases, in particular pulmonary fibrosis.

## Revendications

1. Utilisation d'alprostadil pour la production d'un médicament pour la néogenèse vasculaire dans le cadre du traitement de l'insuffisance cardiaque chronique et/ou de la myocardiopathie.

2. Utilisation selon la revendication 1, **caractérisée en ce que** la néogenèse vasculaire est utilisée pour diminuer le degré de fibrose.

3. Utilisation selon la revendication 1 ou 2, **caractérisée en ce que** la néogenèse vasculaire est utilisée pour la régression de l'hypertrophie.

4. Utilisation selon l'une des revendications 1 à 3, **caractérisée en ce que** la néogenèse vasculaire est utilisée pour la revitalisation de zones cardiaques mortes, en particulier après un infarctus du myocarde.

5. Utilisation selon l'une des revendications 1 à 4, **caractérisée en ce que** la néogenèse vasculaire est utilisée pour le traitement de la maladie artérielle oblitérante périphérique avancée.

6. Utilisation selon l'une des revendications 1 à 5, **caractérisée en ce que** la néogenèse vasculaire est utilisée pour le traitement de l'angiopathie diabétique.

7. Utilisation selon l'une des revendications 1 à 6, **caractérisée en ce que** la néogenèse vasculaire est utilisée pour le traitement de pneumopathies chroniques, en particulier la fibrose pulmonaire.
